# EUROPEAN PATENT APPLICATION

(11) **EP 1 553 189 A1**
(43) Date of publication of application: **13.07.2005**
(21) Application number: 04000343.6
(22) Date of filing: 09.01.2004
(51) Int. Cl.: C12Q 1/68, C12N 15/00

(54) **T7 amplification and cDNA klenow labeling for expression (TAcKLE) analysis with oligonucleotide microarrays**

(71) Applicant: Deutsches Krebsforschungszentrum, 69120 Heidelberg (DE)
(72) Inventor: Thürigen, Dirk Olaf, 69221 Dossenheim (DE); Schlingemann, Jörg, 69221 Dossenheim (DE); Hahn, Meinhard, 34390 Giessen (DE); Lichter, Peter, 69251 Gaiberg (DE)
(74) Representative: VOSSIUS & PARTNER

(57) **Abstract**

The present invention relates to a method of producing labelled, single-stranded antisense cDNA from mRNA, especially useful for hybridization to sense-oriented oligonucleotide libraries. In a preferred embodiment, said producing involves the use of the Klenow fragment of E. coli DNA polymerase I. In another preferred embodiment, the method comprises hybridizing the labelled, single-stranded antisense cDNA to microarrays. Therefore, use of the method for microarray applications, such as comparison of samples obtained from healthy and diseased individuals, is envisaged. Furthermore, a kit for performing the method of the invention is provided.

## Description

The present invention relates to a method of producing labelled, single-stranded antisense cDNA from mRNA, especially useful for hybridization to sense-oriented oligonucleotide libraries. In a preferred embodiment, said producing involves the use of the Klenow fragment of E. coli DNA polymerase I. In another preferred embodiment, the method comprises hybridizing the labelled, single-stranded antisense cDNA to microarrays. Therefore, use of the method for microarray applications, such as comparison of samples obtained from healthy and diseased individuals, is envisaged. Furthermore, a kit for performing the method of the invention is provided.

In this specification, a number of documents is cited. The disclosure of these documents, including manufacturer's manuals, is herewith incorporated by reference in its entirety.

The analysis of mRNA expression of a plurality of genes on DNA chips or microarrays has become a standard method for a broad range of applications. For the quantitative analysis of mRNA expression by hybridization to microarrays, the mRNA to be analyzed has to undergo processing prior to hybridization. Processing aims at one or more of the following: amplification, labelling and/or provision of nucleic acid molecules with the correct orientation.

As regards amplification, it is desirable that the amplification is linear, i.e., the amplification factor does not depend on the amount of the specific nucleic acid species provided in a mixture of different nucleic acids. If this is the case, relative abundances of different nucleic acids remain unchanged and provide a true picture of the relative abundances in the original mRNA sample. Furthermore, as sample material is often limited, it is desirable that said amplification can be accomplished with a high amplification factor.
Labelling is a prerequisite for sensitive detection. For detection via a label it is important that incorporation of the label occurs in an unbiased manner. This is relevant when more than one label, in particular more than one fluorescent dye-labelled nucleotide or nucleotide derivative suited for chemiluminescent or time-resolved chemiluminescent detection measurements, is used. If two or more preparations are labelled with different labels, a preferential incorporation of one label renders a correct assessment of differential expression across the preparations more difficult and prone to systematic error. Furthermore, a high degree of incorporation of the label is desired for achieving maximum sensitivity as well as for economic reasons.

With respect to the orientation of the nucleic acid molecules to be analyzed by hybridizing to a microarray, the specification of the microarray has to be taken into account. While orientation of the nucleic acids to be hybridized is not an issue in case of cDNA microarrays with double-stranded cDNA probes immobilized in their surface, it is evident that orientation matters in case of microarrays with single-stranded oligonucleotide probes or single-stranded cDNA probes which in turn exhibit a specific orientation. In this regard it is important to note that the requirement of correct orientation of the nucleic acid to be hybridized and the incorporation of the label have to be considered together. It is desirable that incorporation of the label occurs preferentially into the nucleic acid molecules with the correct orientation.

A commonly used protocol for mRNA amplification involves in vitro transcription effected with T7 RNA polymerase, originally described by van Gelder et al.¹. The protocol consists of the synthesis of a cDNA complementary to the mRNA ("first strand synthesis"), effected by reverse transcription, followed by second strand synthesis to yield double-stranded cDNA, and in vitro transcription using the double-stranded cDNA as template effected with T7 RNA polymerase. The last step provides single-stranded antisense RNA (aRNA), which may be labelled in case labelled nucleotides are provided. For the entire protocol, the terms "T7 RNA amplification" and "Eberwine protocol" have been coined.
Following the seminal paper by van Gelder et al.¹, variants, improvements and extensions have been developed. Eberwine et al.² extended van Gelder's method by adding a second round of amplification using the RNA obtained in the first round as template.
Wang et al.³ provided a variant of the original T7 method, characterized by a modified second strand synthesis method. The second strand synthesis method of Wang et al. is known in the art as the SMART™ technology (Clontech) for cDNA synthesis. Baugh et al.⁴ describe an optimized variant of the method according to van Gelder et al.¹ and analyze the performance on Affymetrix DNA chips (GeneChip®). Affymetrix Inc. is a commercial provider of high-density oligonucleotide DNA chips (GeneChips®). Affymetrix GeneChips are designed to probe the anti-sense strand. Any other DNA chip or microarray probing the anti-sense strand may be envisaged when performing a T7 RNA amplification, wherein labelling occurs during the in vitro transcription step.

As regards labelling, van Gelder et al. and Eberwine et al. used radioactive labelling by providing a [α-³²P] labelled nucleoside triphosphate for the in vitro transcription step. Aiming to minimize the exposure to radiation, non-radioactive labelling methods have become the method of choice in the meantime. Accordingly, Wang et al. used fluorescent dye-labelled nucleoside triphosphate. Commonly used fluorescent dyes are Cy3 and Cy5. Baugh et al. used biotinylated nucleoside triphosphate (biotin-11-uridine-5'-triphosphate and biotin-11-cytidine-5'-triphosphate). Staining of the biotinylated aRNA was done as specified in the Affymetrix Expression Analysis Technical Manual. Staining according to the Affymetrix Expression Analysis Technical Manual involves adding a streptavidin-phycoerytrin conjugate (SAPE) to the hybridized GeneChip®. The signal may be amplified by subsequently adding biotinylated anti-streptavidin antibody, followed by the addition of SAPE. Amplification of the fluorescent signal may be seen as an additional measure or alternative to amplifying mRNA.

As described above, non-radioactive labelling makes use of nucleotide analogs, i.e., it involves the incorporation of chemically modified nucleotides. In this regard it is important to note that RNA polymerases, as e.g. the T7 RNA polymerase used in the Eberwine protocols, are sensitive with respect to said analogs, i.e., their incorporation occurs with lower preference than that of the naturally occurring unmodified nucleotides.

Smith et al.⁵ describe another amplification protocol, called "single primer amplification (SPA)". It involves first and second strand synthesis as envisaged by the Eberwine protocol, followed by a Taq DNA polymerase amplification step and labelling with Klenow fragment. The amplification is significantly greater than linear. Label is incorporated on both strands, wherein the labelled sense strand is the primary product, and the labelled antisense strand is only a side product.

't Hoen et al.⁶ describe an amplification method according to the Eberwine protocol and provide a comparative analysis of direct incorporation of Cy3/Cy5-labelled UTP during the in vitro transcription step and the incorporation of aminoallyl-UTP with subsequent coupling to Cy3/Cy5 dyes. 't Hoen et al. conclude that the latter approach is characterized by a 2- to 3-fold higher degree of label incorporation and fluorescent signal. Still, the above described sensitivity of RNA polymerases with regard to nucleotide analogs leads to a comparably low degree of aminoallyl-UTP incorporation which requires the use of large amounts of nucleotide analog, thereby rendering the method expensive.

In view of the shortcomings of the methods described in the prior art, the technical problem underlying the present invention therefore was to provide means and methods for the amplification of mRNA, wherein said means and methods yield an amplification product suitable for the hybridization to microarrays or DNA chips with sense-oriented probes immobilized on the surface of said microarrays or DNA chips.

The solution to said technical problem is achieved by providing the embodiments characterized in the claims.

Accordingly, the present invention relates to a method of producing labelled, single-stranded antisense cDNA comprising the steps of (a) creating double-stranded cDNA from mRNA; (b) generating single-stranded antisense RNA from said double-stranded cDNA; (c) generating single-stranded sense cDNA from said single-stranded antisense RNA; and (d) generating labelled, single-stranded antisense cDNA from said single-stranded sense cDNA. Said method is effected by enzymes, which are provided with labelled or unlabelled nucleoside phosphates as building blocks of nucleic acids to be synthesized.

In a preferred embodiment, step (a) comprises the steps (aa) first strand synthesis effected by reverse transcription; and (ab) second strand synthesis effected by DNA polymerization. Reverse transcriptases suitable for step (aa) are known in the art and comprise avian myeloblastoma virus (AMV) reverse transcriptase, Moloney murine leukaemia virus (M-MuLV) reverse transcriptase, ImProm-II reverse transcriptase (ProMega) and Sensiscript reverse transcriptase (Qiagen). Preferably, the reverse transcriptase is Superscript II reverse transcriptase, an engineered version of M-MuLV reverse transcriptase with reduced RNase H activity and increased thermal stability.

In a more preferred embodiment, step (ab) is effected with DNA polymerase I. Preferably, the DNA polymerase is from E. coli.

In a further preferred embodiment, step (b) is effected by in vitro transcription. A number of viral RNA polymerases suitable for in vitro transcription are known in the art. In a more preferred embodiment, said in vitro transcription is effected with an enzyme selected from the group comprising T7 RNA polymerase, T3 RNA polymerase, and SP6 RNA polymerase. Yet more preferred, said enzyme is T7 RNA polymerase.

In a further preferred embodiment, said in vitro transcription is effected under conditions resulting in amplification of said single-stranded antisense RNA. The term "amplification" denotes a process where multiple copies are generated from one or more template molecules. Said conditions are well known in the art and comprise the use of sufficient amounts of labelled and/or unlabelled nucleoside phosphates and RNA polymerase, and/or contacting the template cDNA, the RNA polymerase and the labelled and/or unlabelled nucleoside phosphates for sufficient amount of time.

In a more preferred embodiment, said amplification is linear. The term "linear", when relating to amplification, denotes an amplification method characterized in that the number of copies generated from a template molecule is proportional to the number of template molecules. A linear amplification leaves the relative abundances of distinct molecular species unchanged. In other words, the expression levels or ratios of differential expression within one sample, as determined from the amplified nucleic acids, provide a true picture of the expression levels or ratios of differential expression as determined from the template molecules. The term "ratio of differential expression" denotes the value obtained by diving the expression level of a particular gene (or a number proportional to the expression level) in a first sample by the expression level of the same gene in a second sample. After appropriate normalization, this holds also for expression levels or ratios of differential expression across hybridizations or samples. Therefore, for the accurate quantitative analysis of expression of different mRNA species within one sample and the comparative analysis of expression across different hybridizations and/or samples, a linear amplification offers substantial advantages.

In a yet more preferred embodiment, the amplification step is performed more than once. In this case, reverse transcription (step (aa)), second strand synthesis (step (ab)), and in vitro transcription (step (b)) are performed a second time ("second round of amplification"). The primers used for the second round of amplification are a random hexamer primer for reverse transcription (instead of the oligo-dT + T7 primer of the first round, see also Example 1) and an oligo-dT + T7 primer for second strand synthesis (instead of small fragments resulting from RNase H digestion and serving as primers in the first round). Furthermore, no ligase is used for second strand synthesis in the second round. Thereby, an overall amplification factor of 10⁵ to 10⁶, or even higher, may be attained. The term "overall amplification factor" as used in this specification denotes the amplification factor achieved by performing multiple rounds of amplification. The linear characteristics of the amplification method of the invention, together with the high attainable overall amplification factor permits reliable amplification starting from very small amounts of mRNA. The method of the invention has been tested with 2ng total RNA (corresponding to material obtained from 20 to 30 cells) and was shown to work successfully. It is conceivable that even lower amounts can be amplified faithfully.

In a further preferred embodiment, step (c) of the method of the invention is effected by reverse transcription. Suitable reverse transcriptases are known in the art and comprise avian myeloblastoma virus (AMV) reverse transcriptase, Moloney murine leukaemia virus (M-MuLV) reverse transcriptase, ImProm-II reverse transcriptase (ProMega) and Sensiscript reverse transcriptase (Qiagen). Preferably, the reverse transcriptase is Superscript II reverse transcriptase.

In a further preferred embodiment, step (d) of the method of the invention comprises (da) digestion of the single-stranded antisense RNA; and (db) synthesis of labelled, single-stranded antisense cDNA by DNA polymerization.

In a more preferred embodiment, said digestion is effected either enzymatically with RNase H or chemically with NaOH. Protocols for performing said digestion are known to the skilled person and can be found in Molecular Cloning: A Laboratory Manual (Joseph Sambrook and David W. Russell, CSHL Press).

Most preferred, step (db) of the method of the invention is effected with the Klenow fragment of E.coli DNA polymerase I. E. coli DNA polymerase I is composed of three main domains, i.e., a polymerase domain, a 3'->5' exonuclease domain, and a 5'->3' exonuclease domain. The 3'->5' exonuclease domain is responsible for the proofreading activity of DNA polymerase I, whereas the 5'->3' exonuclease domain is responsible for the degradation of the RNA component of RNA-DNA duplexes. Using protease, e.g. subtilisin, the 5'->3' exonuclease domain can be cleaved off, yielding a fragment commonly referred to as Klenow fragment, which exhibits intact polymerase and 3'->5' exonuclease activity, but, owing to the removal of the 5'->3' exonuclease domain, is permissive with regard to substrates.

The Klenow fragment is particularly suitable for applications where derivatives of natural substrates, e.g. fluorescent dye-labelled substrates or modified substrates suited for chemiluminescence or time-resolved chemiluminescence detection are used. Accordingly, in a further preferred embodiment, the label incorporated into the single-stranded antisense cDNA is a fluorescent label or label suited for conventional or time-resolved chemiluminescence detection. In a more preferred embodiment, said label is a fluorescent deoxyribonucleotide or nucleotide derivative suited for conventional or time-resolved chemiluminescence detection.
An enzyme which uses naturally occurring nucleotides and fluorescent dye-labelled nucleotides with substantially the same preference is desirable, as thereby an efficient use of the labelled nucleotides provided in the medium is ensured.

Labelling methods other than the direct incorporation of fluorescent labels are also envisaged. Said methods comprise the incorporation of aminoallyl-nucleoside triphosphates as described for example by 't Hoen et al.⁶ and the incorporation of biotinylated nucleoside triphosphates as for example described by Baugh et al.⁴. Alternatively, chemiluminescence-based detection schemes known in the art ^{7,8} may be used.

As reviewed in reference 9, time-resolved fluorometry utilizes the long-lived fluorescence of rare earth metals (lanthanides). The fluorescence of lanthanides, e.g. europium (Eu), terbium (Tb), Samarium (Sm), can be measured after a time delay when the short-lived background fluorescence of biological material has decayed. In addition, the emission peaks of different lanthanides are clearly distinguishable because they are sharp and occur at different wavelengths, making the simultaneous measurement possible. Chelates of lanthanides can be used as labels in various applications without changing the properties of bioaffinity molecules.

The Eberwine protocol and its variants known in the art (references 1 to 4) perform labelling with the in vitro transcription step. The RNA polymerases employed for in vitro transcription are significantly less tolerant with regard to chemically modified substrates as compared to the Klenow fragment. Therefore, the method of the invention offers significant advantages over the Eberwine protocol and its variants.

Alternatively, labelling is done by reverse transcription from the antisense RNA obtained by in vitro transcription (see Wang et al.³). In this case, the resulting cDNA is sense-oriented and therefore incompatible with hybridization to microarrays or DNA chips with sense probes.

Smith et al.⁵ use Klenow fragment for labelling. However, their methods yields predominantly labelled sense strand, which is not suitable for analysis on microarrays or DNA chips with sense-oriented probes. On the other hand, the labelled product of the method of the invention is only antisense cDNA. Smith et al.⁵ furthermore state that their amplification method is non-linear, i.e., it does not exhibit the fidelity with regard to relative mRNA abundances of the method of the invention.

A further advantage of the method of the invention is that the labelled product is DNA, whereas in vitro transcription yields RNA. As DNA is significantly more stable and more resistant to hydrolysis than RNA, handling is less cumbersome.

A yet further advantage of the Klenow fragment pertains to applications where more than one type of label is used. Owing to its tolerance regarding chemically modified substrates, the use of Klenow fragment for labelling does not introduce a label-dependent bias. Such bias would occur, if one label is incorporated with greater preference than another label. Accordingly, in a further preferred embodiment of the method of the invention, at least two different fluorescent labels or labels suited for conventional or time-resolved chemiluminescence detection are used. In a more preferred embodiment, said two fluorescent labels or labels suited for conventional or time-resolved chemiluminescence detection are incorporated in the same ratio or substantially the same ratio as they are provided in the medium. This applies both to cases where more than one label is used in one experiment as well as to cases where different labels are used in different experiments.

The DNA polymerization step can be designed such that amplification or further amplification in addition to amplification by the in vitro transcription step, respectively, takes place. This is desirable in particular in those cases where little sample material is available. Accordingly, said DNA polymerization is effected under conditions resulting in the amplification of said single-stranded antisense cDNA. Such conditions are well known in the art and comprise the use of sufficient amounts of labelled and/or unlabelled nucleoside phosphates and DNA polymerase, and/or contacting the template cDNA, the DNA polymerase and the labelled and/or unlabelled nucleoside phosphates for sufficient amount of time.

Any source of the starting mRNA material is envisaged. In a preferred embodiment, said mRNA is comprised in a sample obtained from cell lines or tissue probes or single cells.

Said mRNA may be provided in different forms, said forms comprising total RNA and purified mRNA. The term "total RNA" denotes the complete RNA content of a sample, as obtained by procedures known in the art, e.g. phenol guanidinium isothiocyanate extraction (Trizol). Procedures for the enrichment and/or purification of mRNA are known in the art. Certain procedures exploit the fact that mature mRNAs are polyadenylated at the 3' end. For example, oligo(dT)-cellulose chromatography may be employed. The obtained mRNA preparation is commonly referred to as poly(A)⁺ RNA, thereby indicating the presence of the poly(A) tail at the 3' end.

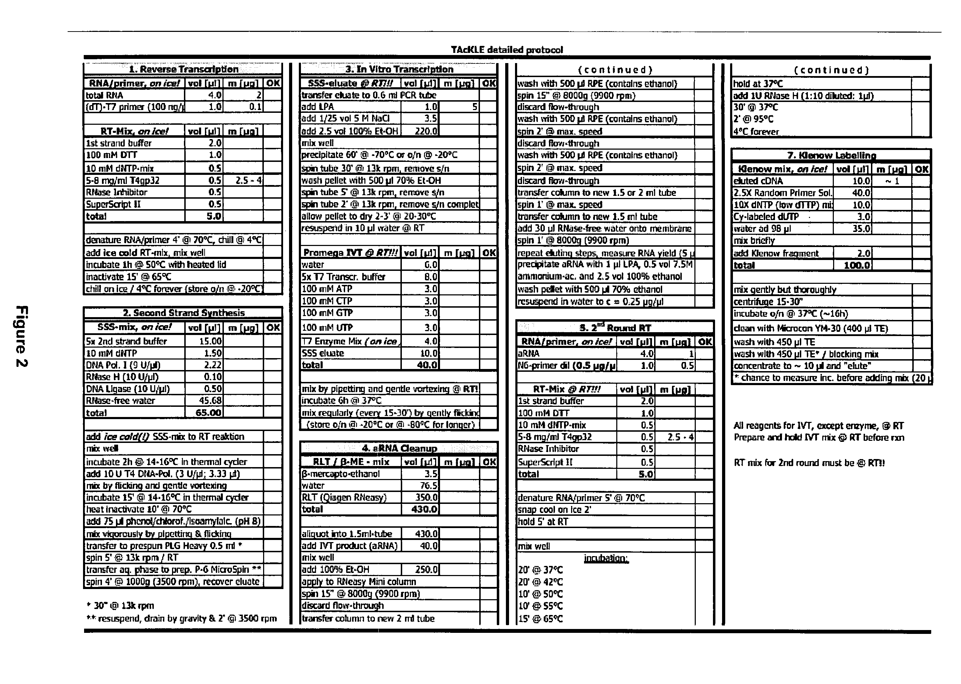

Exemplary protocols for performing the method of the invention are provided in Example 1 and Figure 2.

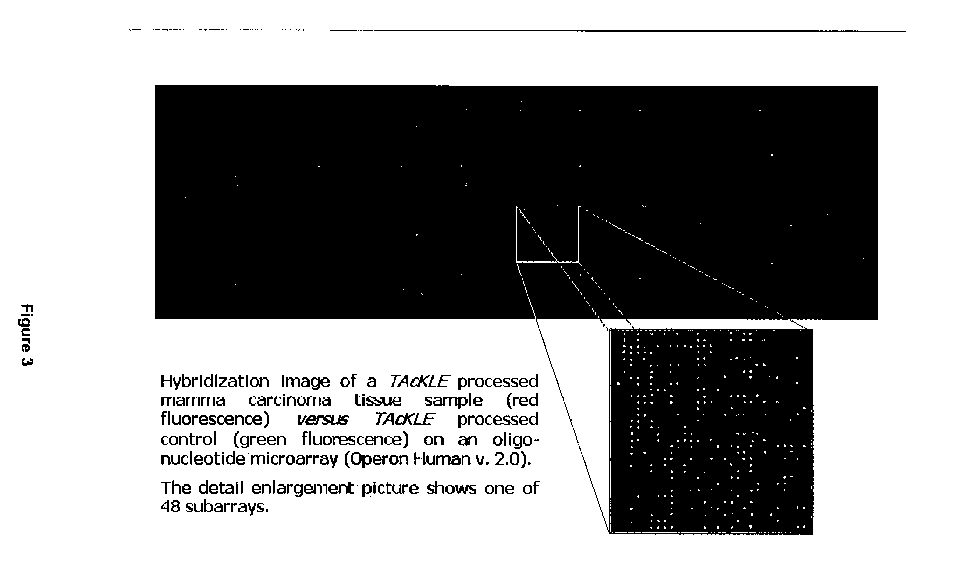

An important application of the method of the invention involves the quantitative analysis of the expression of mRNA on microarrays or DNA chips. Therefore, in a preferred embodiment, the further step of hybridizing the labelled, single-stranded antisense cDNA to one or more microarray(s) or DNA chip(s) is envisaged, wherein the probes immobilised on the surface of said microarray or DNA chip are single-stranded sense DNA oligonucleotides, single-stranded sense cDNAs or double-stranded cDNAs. Microarrays and DNA chips are devices comprising a supporting material (a filter or a solid support such as glass) and probes immobilized on the supporting material and arranged in arrayed form. Figure 3 shows an example of a hybridized oligonucleotide microarray, wherein the mRNA has been amplified with the method of the invention.

In a more preferred embodiment, said hybridizing is specific. Means of ensuring specificity of hybridization according to the present invention are known in the art and include stringent hybridization conditions. The term "stringent hybridization conditions" is well known to the skilled artisan. Appropriate stringent hybridization conditions for each sequence may be established by a person skilled in the art on well-known parameters such as temperature, length and composition of the nucleic acid molecules, salt conditions etc. Specifically, the extent of hybridization depends on the melting temperature of the nucleic acid molecules involved. The melting temperature in turn depends on the sequence length (and composition). The simplest rule for the estimation of the melting temperature is the Wallace rule (Wallace et al., Nucleic Acids Res. 6, 3543-3557 (1979)), which uses a linear relation between the number of nucleotides with base-specific weightings. The skilled person is aware of this and related methods and thus knows how to determine stringent hybridization conditions in dependency of the sequence length; see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1x SSC at about 65°. Other stringent hybridization conditions are for example 0.2 x SSC (0.03 M NaCl, 0.003M Natriumcitrat, pH 7) at 65°C.

Amplification protocols known in the art, including the protocols described in references 5 and 6, result in differential expression ratios that are compressed in comparison with the original expression ratios determined from non-amplified material. The term "compressed" in relation to ratios of differential expression denotes ratios which are smaller as compared to a standard of truth. A suitable standard of truth, for example, are ratios determined from unamplified mRNA in those cases where sufficient amounts of mRNA are available. Surprisingly, differential expression ratios as determined with the labeled, single-stranded antisense cDNA obtained from mRNA with the method of the invention, are not compressed. Therefore, in a more preferred embodiment it is envisaged that at least two different samples comprising said mRNA are obtained and the differential expression ratios between said samples are not compressed.

The microarray technology is being employed or can be employed for many applications. Just to name one example, microarrays are used for the comparative analysis of tumor samples vs. samples derived from healthy tissue(s). It is envisaged that the method of the invention is used for processing the sample prior to hybridization to microarrays or DNA chips. In conjunction with the above described lanthanide-based detection method, the parallel comparative analysis of three or more samples is envisaged.

The present invention furthermore relates to a kit comprising (a) a reverse transcriptase selected from the group consisting of Superscript II reverse transcriptase, avian myeloblastoma virus (AMV) reverse transcriptase, Moloney murine leukaemia virus (M-MuLV) reverse transcriptase, ImProm-II reverse transcriptase (ProMega) and Sensiscript reverse transcriptase (Qiagen); (b) E. coli DNA polymerase I; (c) an RNA polymerase selected from the group consisting of T7 RNA polymerase, T3 RNA polymerase, or SP6 RNA polymerase; (d) optionally RNase H; (e) Klenow fragment of E.coli DNA polymerase I; and (f) a prescription for performing the method of any one of the preceding claims.

The Figures show:

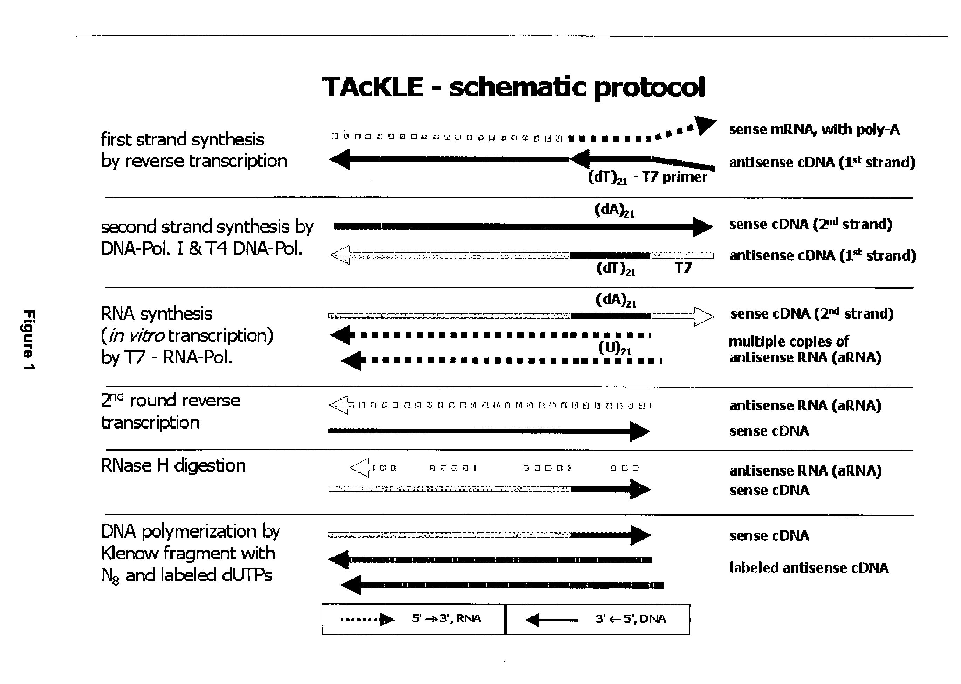

**Figure 1.** Schematic representation of an example of the *TAcKLE* protocol.
**Figure 2.** Detailed example of the *TAcKLE* protocol.
**Figure 3.** Hybridization image of a *TAcKLE* processed mamma carcinoma tissue sample versus a *TAcKLE* processed control on an oligo-nucleotide microarray.
**Figure 4.** Validation of the *TAcKLE* protocol for microarray experiments.

The Examples illustrate the invention.

### Example 1

### Materials and Methods

For amplification and labeling using the *TAcKLE* protocol, 2 µg total RNA were employed in first- and second-strand cDNA synthesis as previously described (Baugh LR *et al.,* 2001), with minor modifications. Shortly, RNA was mixed with 100 ng (dT)-T7 primer (5'- GCA TTA GCG GCC GCG AAA TTA ATA CGA CTC ACT ATA GGG AGA (T)₂₁ VN - 3'; Biospring, Frankfurt, Germany) to a final volume of 5 µl, denatured 4 min at 70 °C and chilled on ice. 5 µl ice-cold RT mix were added to the samples, yielding final concentrations of 1x First-Strand Buffer (Invitrogen, Karlsruhe, Germany), 10 mM dithiothreitol (DTT; Invitrogen), 500 µM dNTP (Amersham Biosciences, Freiburg, Germany), 400 ng/µl T4gp32 (USB, Cleveland, OH, USA), 2 U/µl RNasin ribonuclease inhibitor (Promega, Mannheim, Germany) as well as 10 U/µl Superscript II reverse transcriptase (Invitrogen). Reverse transcription was performed 1 h at 50 °C and reactions were stopped by heating to 65 °C for 15 min. Following the addition of 65 µl ice-cold SSS mix, second-strand synthesis was performed for 2 h at 15 °C in 1 x Second-Strand buffer (Invitrogen), 200 µM dNTP, 0.27 U/µl DNA polymerase I (Promega), 1 U RNase H (Epicentre, Madison, WI, USA) and 5 U *E. coli* DNA ligase (Amersham Biosciences). Then, 10 U T4 DNA polymerase (NEB, Beverly, MA, USA) were added to the samples and cDNA ends were polished for 15 min at 15 °C. Enzymes were inactivated by 10 min incubation at 70 °C. To extract double-stranded cDNA, samples were mixed with 75 µl phenol / chloroform / isoamylalcohol (pH 8.0) (Sigma-Aldrich, Munich, Germany) and transferred to prespun 0.5 ml PLG heavy tubes (Eppendorf, Hamburg, Germany). After 5 min centrifugation at 13,000 rpm, the aqueous phase was further purified on a P-6 Micro BioSpin column (Bio-Rad, Munich, Germany) according to the manufacturer's instructions, followed by ethanol precipitation. cDNA was dissolved in 10 µl nuclease-free water and employed in an *in vitro* transcription reaction using a RiboMAX Large Scale RNA Production Kit (T7) (Promega) according to the manufaturer's recommendations, but scaling the reaction volume up to 40 µl and regularly mixing the tubes every 30 min for a total reaction time of 6 h. Following purification on RNeasy Mini filters (QIAGEN, Hilden, Germany) and ethanol precipitation, aRNA was dissolved in 4 - 10 µl nuclease-free water. Second round reverse transcription was performed on 1 µg aRNA as described above, except for the following modifications: 0.5 µg random hexamer primer (Roche Diagnostics, Mannheim, Germany) were used instead of (dT)-T7 primer, samples were incubated 2 min at room temperature before addition of RT mix to allow for annealing of N6-primer, and the following temperature profile was employed: 20 min at 37 °C, 20 min at 42 °C, 10 min at 50 °C, 10 min at 55 °C, 15 min at 65 °C. RNase H digestion (1 U) was carried out for 30 min at 37 °C, followed by 2 min at 95 °C to degrade enzymes. cDNA labeling by Klenow fragment was performed using the Bioprime Kit (Invitrogen), but with a modified protocol. Shortly, 10 µl cDNA sample were mixed with 40 µl random primer solution, 10 µl 10 x dNTP mix (2 mM dATP, dCTP, dGTP; 0.5 mM dTTP), 3 µl Cy-dUTP (1 mM; Amersham Biosciences) and ddH₂O ad 98 µl. Following the addition of 2 µl high concentration Klenow fragment (40 - 50 U/µl), samples were incubated at 37 °C for 16 h.

### Example 2

### Validation of the TAcKLE protocol for microarray experiments

Samples from two different cell lines have been prepared according to different protocols and hybridized to oligonucleotide microarrays. The following protocols have been applied: (a) the *TACKLE* protocol of the present invention, (b) the single primer amplification protocol according to Smith et al.⁵, (c) *in vitro* transcription according to Baugh et al.⁴, and (d) no amplification, i.e. unamplified RNA has been used. The cell lines used were HL-60 and SU-DHL-1. The oligonucleotide microarrays were Operon Human v. 2.0 microarrays. This microarray comprises a commercial library of 21329 bioinformatically optimized, transcript specific oligonucleotides immobilized on an epoxysilane-coated glass slide.

Upon hybridization and data acquisition, the correlation of two samples of identical cell lines or two different cell lines, respectively, processed according to the above mentioned protocols, has been determined.

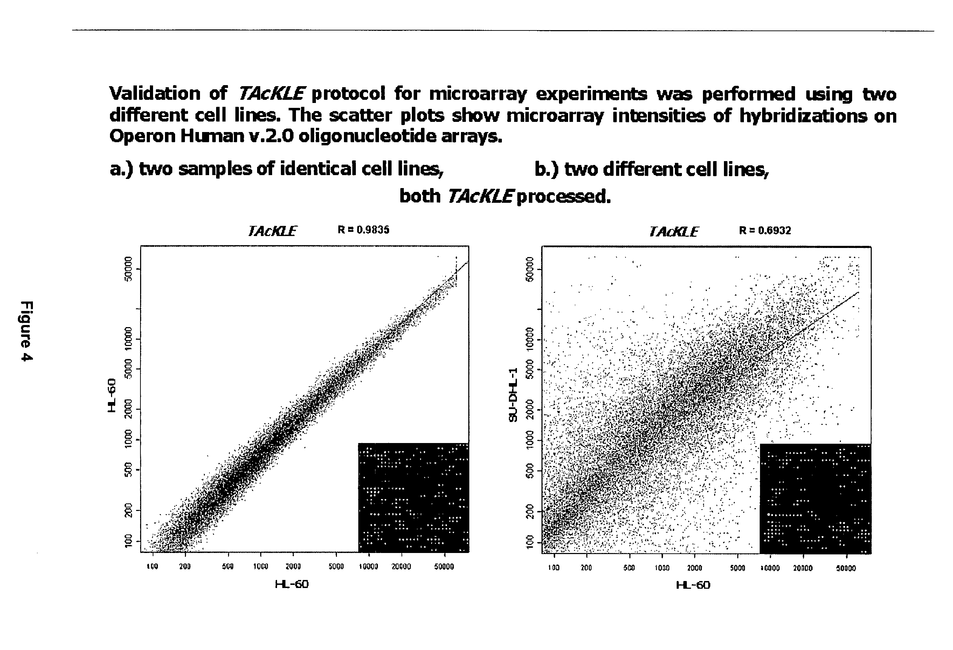

Figure 4 shows two examples of scatterplots. The scatterplot on the left (a) shows the correlation between two microarray experiments performed on two samples derived from the same cell line, both *TAcKLE* processed.

In summary, the *TAcKLE* protocol is characterized by superior amplification rates, high correlation between identical experiments, accordingly high reproducibility and fidelity.

### Example 3

### Classification of patients by their expression profile

One of the aims of expression profiling using DNA microarrays is the classification of patients. Patients frequently differ significantly with regard to course of disease and response to treatment.

Tissue sample material has been analyzed with regard to a possible gene expression signature capable of classifying patients according to their different clinical response.

Clustering analysis of data obtained from DNA oligonucleotide microarrays shows that the *TAcKLE* amplification method allows discrimination of different patient RNA samples according to their clinical response to the treatment. The fidelity of amplification of the method hereby proves to be high enough to identify gene expression signatures which are comparable with those observed with unamplified RNA, while using 100 to 1000-fold smaller amounts of RNA.

### References

1. van Gelder, R. N., von Zastrow, M. E., Yool, A., Dement, W. C., Barchas, J. D. and Eberwine, J. H. Amplified RNA synthesized from limited quantities of heterogeneous cDNA. *Proc. Natl. Acad. Sci. USA* **87,** 1663-1667 (1990).
2. Eberwine, J., Yeh, H., Miyashiro, K., Cao, Y., Nair, S., Finnell, R., Zettel, M. and Coleman, P. Analysis of gene expression in single live neurons. *Proc. Natl. Acad. Sci. USA* **89,** 3010-3014 (1992).
3. Wang, E., Miller, L. D., Ohnmacht, G. A., Liu, E. T. and Marincola, F. M. High-fidelity mRNA amplification for gene profiling. *Nature Biotechnol.* **18,** 457-459 (2000).
4. Baugh, L. R., Hill, A. A., Brown, E. L. and Hunter, C. P. Quantitative analysis of mRNA amplification by in vitro transcription. *Nucleic Acids Res.* **29,** E29 (2001).
5. Smith, L., Underhill, P., Pritchard, C., Tymowska-Lalanne, Z., Abdul-Hussein, S., Hilton, H., Winchester, L., Williams, D., Freeman, T., Webb, S. and Greenfield, A. Single primer amplification (SPA) of cDNA for microarray expression analysis. *Nucleic Acids* Res. **31,** E9 (2003).
6. 't Hoen, P. A. C., de Kort, F., van Ommen, G. J. B. and den Dunnen, J. T. Fluorescent labelling of cRNA for microarray applications. *Nucleic Acids Res.* **31**, E20 (2003).
7. Heinonen, P., litia, A., Torresani, T. and Lovgren, T. Simple triple-label detection of seven cystic fibrosis mutations by time-resolved fluorometry. *Clin Chem.* **43,** 1142-1150 (1997).
8. Wu, F. B., Han, S.Q., Xu, T. and He, Y.F. Sensitive time-resolved fluoroimmunoassay for simultaneous detection of serum thyroid-stimulating hormone and total thyroxin with Eu and Sm as labels. *Anal. Biochem.* **314,** 87-96 (2003).
9. Dickson, Pollak, A. and Diamandis, E.P. Ultrasensitive bioanalytical assays using time-resolved fluorescence detection. *Pharmacol. Ther.* **66,** 207-235 (1995).

## Claims

1. A method of producing labelled, single-stranded antisense cDNA comprising the steps of:
(a) creating double-stranded cDNA from mRNA;
(b) generating single-stranded antisense RNA from said double-stranded cDNA;
(c) generating single-stranded sense cDNA from said single-stranded antisense RNA; and
(d) generating labelled, single-stranded antisense cDNA from said single-stranded sense cDNA.

2. The method according to claim 1, wherein step (a) comprises the steps of:
(aa) first strand synthesis effected by reverse transcription; and
(ab) second strand synthesis effected by DNA polymerization.

3. The method according to claim 2, wherein step (ab) is effected with E. coli DNA polymerase I.

4. The method according to any one of claims 1 to 3, wherein step (b) is effected by in vitro transcription.

5. The method according to claim 4, wherein said in vitro transcription is effected with an enzyme selected from the group comprising T7 RNA polymerase, T3 RNA polymerase, and SP6 RNA polymerase.

6. The method according to claim 4 or 5, wherein said in vitro transcription is effected under conditions resulting in amplification of said single-stranded antisense RNA.

7. The method according to claim 6, wherein said amplification is linear.

8. The method according to claim 6 or 7, wherein the amplification step is performed more than once.

9. The method according to any one of claims 1 to 8, wherein step (c) is effected by reverse transcription.

10. The method according to any one of claims 1 to 9, wherein step (d) comprises
(da) digestion of the single-stranded antisense RNA; and
(db) synthesis of labelled, single-stranded antisense cDNA by DNA polymerization.

11. The method according to claim 10, wherein step (da) is effected with RNase H or NaOH.

12. The method according to claim 10 or 11, wherein step (db) is effected with the Klenow fragment of E.coli DNA polymerase I.

13. The method according to any one of claims 10 to 12, wherein said DNA polymerization is effected under conditions resulting in the amplification of said single-stranded antisense cDNA.

14. The method according to any one of claims 1 to 13, wherein said mRNA is comprised in a sample obtained from cell lines or tissue probes or single cells.

15. The method according to any one of claims 1 to 14, wherein said mRNA is provided as total RNA or as purified mRNA.

16. The method according to any one of claims 1 to 15, wherein the label is a fluorescent label or label suited for conventional or time-resolved chemiluminescence detection.

17. The method according to any one of claims 1 to 16, wherein said label is a fluorescent deoxyribonucleotide or nucleotide derivative suited for conventional or time-resolved chemiluminescence detection.

18. The method according to any one of claims 1 to 17, wherein at least two different fluorescent labels or labels suited for conventional or time-resolved chemiluminescence detection are used.

19. The method according to claim 18, wherein said two fluorescent labels or labels suited for conventional or time-resolved chemiluminescence detection are incorporated in the same ratio as they are provided in the medium.

20. The method according to any one of claims 1 to 19, comprising the further step of hybridising the labelled, single-stranded antisense cDNA to one or more microarray(s) or DNA chip(s), wherein the probes immobilised on the surface of said microarray or DNA chip are single-stranded sense DNA oligonucleotides, single-stranded sense cDNAs or double-stranded cDNAs.

21. The method according to claim 20, wherein at least two different samples comprising said mRNA are obtained and the differential expression ratios between said samples are not compressed.

22. A kit comprising
(a) a reverse transcriptase selected from the group consisting of Superscript II reverse transcriptase, avian myeloblastoma virus (AMV) reverse transcriptase, Moloney murine leukaemia virus (M-MuLV) reverse transcriptase, ImProm-II reverse transcriptase and Sensiscript reverse transcriptase;
(b) E. coli DNA polymerase I;
(c) an RNA polymerase selected from the group consisting of T7 RNA polymerase, T3 RNA polymerase, or SP6 RNA polymerase;
(d) optionally RNase H;
(e) Klenow fragment of E. coli DNA polymerase I; and
(f) a prescription for performing the method of any one of the preceding claims.
